# EUROPEAN PATENT APPLICATION

(11) **EP 4 509 210 A2**
(43) Date of publication of application: **19.02.2025**
(21) Application number: 24212167.1
(22) Date of filing: 28.07.2017
(51) Int. Cl.: B01J 13/00

(54) **PROPELLANTS AND EMULSIFICATION COMPOSITIONS**

(30) Priority: 28.07.2016 US 201662368001 P
(62) Divisional of application: 17835396.7
(71) Applicant: Honeywell International Inc., Morris Plains NJ 07950 (US)
(72) Inventor: PARVATIYAR, Madan, Williamsville, 14221 (US)
(74) Representative: Stepney, Gregory John

(57) **Abstract**

Disclosed is a stable micro-emulsion comprising: water; a water soluble, hydroxy-containing polymeric material; (c) at least one a hydrotrop; and (d) a propellant comprising trans-1,3,3,3-tetrafluoropropene.

## Description

### CROSS REFERENCE TO RELATED APPLICATIONS

The present application is related to and claims the priority benefit of U.S. Provisional Application No. 62/368,001, filed July 28, 2016, which is incorporated herein by reference.

### FIELD

The present invention relates to micro-emulsions. More particularly, it relates to the aqueous microemulsions, and to methods of forming aqueous microemulsions and to compositions and products that contain aqueous microemulsions.

### BACKGROUND

Microemulsions have heretofore been known. One example is an oil-in-water microemulsion as set forth in U.S. 4,146,499. As explained in U.S. 4,146,499, microemulsions as previously known were dispersions of two immiscible liquids (one liquid phase being "dispersed" and the other liquid being "continuous") in which the individual liquid droplets of the dispersed phase have an average radius less than about 1/4 of the wavelength of light. Typically, in a microemulsion that have heretofore been made the dispersed phase liquid droplets are less than about 1,400A radius, and preferably in the order of 100A to 500A. In such a case, even though the second liquid is not in technically in solution, the droplets are sufficiently small that they do not disperse light and therefore are essentially transparent. This patent describes a relatively complex process for making such microemulsions. The disclosed process involves a four-step process that uses at least two surfactants, a material that makes up the oil phase of the oil-in-water emulsion, and a specific order for combining the various ingredients that requires that before titration with the secondary solvent the oil-in-water system must first be converted into a finely divided lactescent emulsion.

US Patent No. 4,536,323 describes an improvement on the oil-in-water type of microemulsions described in U.S. 4,146,499. The microemulsions disclosed in US Patent No. 4,536,323 comprise a continuous aqueous phase and a disperesed oil phase. The preferred oil phase is methylene chorfide, which is disclosed to be present in relatively large concentrations (23 - 35% by weight). These microemulsions include a material which is described as a propellant which is said to be dissolved the oil-phase. Numerous ionic and non-ionic surfactants are disclosed for use in the oil-in-water type of microemulsions described in U.S. 4,146,499.

Applicants have come to appreciate that significant disadvantages are associated with micro-emulsions of the type described above. For example, oil-in-water microemulsions as set forth in U.S. 4,146,499 requires a relatively omplex procedure to form the microemulsion, and the microemulsion of US Patent No. 4,536,323 requires the formation of an oil-in-water microemulsion based on the use of relatively large amount of an oil that is immiscible in water, such as methylene chloride, which entails handling and processing of large amounts of materials that it would be desirable to avoid. In particular with respect to methylene chloride, OSHA considers methylene chloride to be a potential occupational carcinogen, with short-term exposures to high concentrations potentially causing mental confusion, lightheadedness, nausea, vomiting, and headache.

Furthermore, the propellants disclosed in US Patent No. 4,536,323 are mostly hydrocarbons, which are flammable and therefore present difficulties and potential risks, even though the microemulsion itself is said to be nonflammable. Other materials that might have been considered for use as a propellant, such as certain chlorofluorocarbons (CFCs) have a harmful effect on the Earth's ozone layer. Since the ozone layer filters harmful radiation from the Earth's surface, increased incidences of skin cancer are believed to result from reductions in the ozone layer thickness or concentration.

Certain saturated hydrofluorocarbons (HFCs) have been used to replace CFC compounds in many instances. HFC compounds do not deplete the ozone, but it has recently been discovered that they can have an impact on global warming. Many HFC compounds are more potent warming agents than carbon dioxide. For example, HFC-134a (1,1,1,2-tetrafluoroethane), which has an ODP of zero, has been used as a propellant in aerosols, but has a high global warming potential (GWP) of 1300.

Applicants have therefore recognized that a need exists to formulate microemulsions that overcome one or more of the disadvantages described above and which utilize propellants that have a low ozone depletion potential, low global warming potential, and thereby overcome one or more of the disadvantages of the propellants described above.

### SUMMARY

In order to address and overcome one or more of the disadvantages described above, as well others, applicants have developed a new type of aqueous-based microemulsion that includes a dispersed, non-flammable propellant and which does not require the use of an oil as a dispersed phase in a continuous aqueous phase in order to dissolve the propellant.

One preferred embodiment of the present invention provides an aqueous micro-emulsion that comprises:
a. an aqueous phase;
b. a water soluble, hydroxy-containing polymeric material, preferably comprising a polyalkyleneglycol, and even more preferably polyethylene glycol (PEG) and/or polypropyleneglycol (PPG);
c. at least one hydrotrop; and
d. a propellant phase comprising trans-1,3,3,3-tetrafluoropropene (HFC-1234ze(E) or transHFO-1234ze), wherein said propylene glycol and said at least one hydrotrop are present in amounts effective to create a stable microemulsion of said propellant in said aqueous phase or of said aqueous phase in said propellant.

The preferred stable, aqueous microemulsions of the present invention are unexpected since the preferred propellants are not soluble in water and since an oil phase is not required in order to create a disperse phase into which the propellant can be solvated according to the preferred aspects of the present invention.

As used herein, the term "aqueous microemulsion" means an emulsion which either has a continuous phase comprising water and a disperse phase comprising propellant or a continuous phase comprising propellant and a disperse phase comprising water.

As used herein, the terms "microemulsion," "microemulsified" and the like mean a stable, essentially transparent liquid, that is, a liquid which gives the appearance to the unaided eye of being a single phase.

As used herein, the term "stable" means the microemulsion remains essentially transparent and giving the appearance of one phase for at least a period of six weeks when stored under ambient temperature (i.e., room temperature) conditions, more preferably in which it is stored when stored under elevated temperature (i.e., 40°C or 50°C) conditions for six weeks, and even more preferably when stored under ambient temperature (i.e., room temperature) conditions for one year. In certain highly preferred embodiments, the microemulsion of the present invention remains essentially transparent and giving the appearance of one phase for at least a period of two years when stored under ambient temperature (i.e., room temperature) conditions. Preferrably, the microemulsion of the present invention remains essentially transparent and giving the appearance of one phase for the entire time from its creation to the time of expected use. Preferrably, the microemulsion of the present invention exhibits no Ostwald ripening during at least six months, more preferably at least one year, and even more preferably for at least two years when stored at ambient temperature conditions.

As used herein, the term "contains no substantial oil phase" means that the microemulsion contains not more than 5% by weight of a component other than said propellant that is essentially immiscible in water under ambient temperature conditions and more preferably not more than 2.5% of such a component, and even more preferably not more than 1% of such a component.

Accordingly, a preferred embodiment of the present invention provides an aqueous micro-emulsion that comprises:
a. a continuous aqueous phase;
b. a water soluble, hydroxy-containing polymeric material, preferably comprising a polyalkyleneglycol, and even more preferably polyethylene glycol (PEG) and/or polypropyleneglycol (PPG);
c. at least one hydrotrop; and
d. a propellant comprising trans-1,3,3,3-tetrafluoropropene (HFC-1234ze(E) or transHFO-1234ze) microemulsified in said aqueous phase, wherein said polypropylene glycol and said at least one hydrotrop are present in amounts effective to create a stable microemulsion of said propellant in said aqueous phase, and wherein said aqueous microemulsion contains no substantial amount of an oil phase dispersed in said aqueous continuous phase.

According to a preferred embodiment of the present invention, an aqueous micro-emulsion is provided that comprises:
a. a continuous propellant phase, said propellant comprising trans-1,3,3,3-tetrafluoropropene (HFO-1234ze(E) or transHFO-1234ze);
b. a water soluble, hydroxy-containing polymeric material, preferably comprising a polyalkyleneglycol, and even more preferably polyethylene glycol (PEG) and/or polypropyleneglycol (PPG);
c. at least one hydrotrop; and
d. an aqueous disperse phase comprising water microemulsified in said propellant phase, wherein said propylene glycol and said at least one hydrotrop are present in amounts effective to create a stable microemulsion of said aqueous phase in said propellant, and wherein said aqueous microemulsion contains no substantial amount of an oil phase dispersed in said aqueous continuous phase.

According to a preferred embodiment of the present invention, an aqueous micro-emulsion is provided that comprises:
a. a continuous propellant phase, said propellant comprising trans-1,3,3,3-tetrafluoropropene (HFO-1234ze(E) or transHFO-1234ze);
b. a water soluble, hydroxy-containing polymeric material, preferably comprising a polyalkyleneglycol, and even more preferably polyethylene glycol (PEG) and/or polypropyleneglycol (PPG);;
c. at least one hydrotrop; and
d. an aqueous disperse phase comprising water microemulsified in said propellant phase, wherein said propylene glycol and said at least one hydrotrop are present in amounts effective to create a stable microemulsion of said aqueous phase in said propellant, and wherein said aqueous microemulsion contains no substantial amount of an oil phase.

The present invention also provides a functional composition comprising at least one active component contained in the microdispersion of the present invention.

The present invention also provides methods of forming microemulsions.

The present invention also provides devices and articles containing a microemulsion and/or a functional composition of the present invention, preferably under pressure, and a nozzle, orifice, valve and the like in, on or in association with the container for dispensing the microemulsion and/or a functional composition of the present invention to an area or surface of intended use. Preferably, the present invention provides methods for dispensing microemulsion and/or a functional composition to an area or surface of intended use wherein said propellant provides at least a portion of the motive force to move said microemulsion and/or a functional composition out of the container through said nozzle, orifice, valve and the like.

### DETAILED DESCRIPTION

### THE MICROEMULSIONS

As mentioned above, the present invention provides in one embodiment a microdispersion comprising the following ingredients: an aqueous phase; a propellant phase; a water soluble polymer; a hydrotrop; optionally isopropanol; and optionally glycerin. Preferred characteristics, properties and structures for each of these are described below:

### The Aqueous Phase

As mentioned above, the aqueous phase of the present invention can exist as the disperse phase or the continuous phase, and in general will comprise water. It will be appreciated, however, that numerous other components can be incorporated in and/or associated with the aqueous phase. For example, at least some portion of the water soluble, hydroxy-containing polymeric material may be contained within the aqueous phase, but in general it is believed and preferred that some portion of the preferred water soluble, hydroxy-containing polymeric material may also be located in the propellant phase and/or at the interface between the phases. Accordingly, all such arrangements and combinations of arrangements are with the scope of the present invention.

### The Propellant

The propellant of the present invention comprises the hydrofluoroolefin (HFO) tetrafluoropropene (HFO-1234). The term "HFO-1234" is thus used herein to refer to tetrafluoropropenes. Preferred among the tetrafluoropropenes are 2,3,3,3-tetrafluoropropene (HFO-1234yf) and both cis- and trans-1,3,3,3-tetrafluoropropene (HFO-1234ze). The term HFO-1234ze is used herein to refer to 1,3,3,3-tetrafluoropropene, independent of whether it is the cis- or trans-isomer. The terms "cis HFO-1234ze" and "trans HFO-1234ze" are used herein to describe the cis- and trans-isomers of 1,3,3,3-tetrafluoropropene respectively. The term "HFO-1234ze" therefore includes cis HFO-1234ze, trans HFO-1234ze, and all combinations and mixtures of these. HFO-1234 is commercially available from Honeywell International and is described in U.S. Patent Publication 2008/0292564, which is incorporated by reference herein in its entirety.

The propellant may comprise a single isomer of HFO-1234 (notwithstanding the presence of trace impurities of other HFO-1234 isomers), or a mixture of HFO-1234 isomers. In preferred embodiments the HFO-1234ze comprises at least about 90 %, at least about 95%, at least about 97%, at least about 99%, at least about 99.5% of transHFO-1234ze based on the total of the cis and trans isomers of HFO-1234ze.

In a preferred embodiments, the propellant comprises trans HFO-1234ze. Preferably the propellant comprises transHFO-1234ze in an amount of at least about 70 % by weight based on the total propellant. In other embodiments, the propellant comprises at least about 75 %, at least about 80 %, at least about 85 %, at least about 90 %, at least about 95 %, at least about 97%, at least about 98.5%, at least about 99.5% by weight based on the propellant. In a further embodiment, the propellant consists essentially of transHFO-1234ze. In a further embodiment, the propellant consists of transHFO-1234ze.

The propellant may further comprise, in addition to the preferred HFO-1234 or or more preferred HFO-1234ze, and even more preferred trans HFO-1234ze, one or more additional propellants. Such additional propellants can include, for example, HFC-152a, HFC-134a, HFC-227ea, propane, butane, isobutane, CO₂, N₂, dimethyl ether, and combinations thereof. An exemplary combination is trans HFO-1234ze and HFC-134a.

The propellant can be present in a wide range of concentrations of the microemulsion of the present invention. In preferred embodiments, the propellant is present an amount of from about 5 weight percent to about 90 weight percent, based on the total weight of the present microemulsion, including in the form of an aerosol spray composition, and more preferably from about 10 to about 50 weight percent, and more preferably from about 10 to about 30 weight percent of the composition. Within this range the propellant can be present in an amount greater than about 10 weight percent. Also within this range the propellant can be present in an amount less than about 80 weight percent.

The HFO-1234 propellant is insoluble with water and immediately separates into a separate phase upon mixing with water. Applicants have unexpectedly found that notwithstanding this problem it is unexpectedly possible to make a microemulsion, which provides a clear, stable mixture of this components, and that such microemulsions according to the present invention are advantageous for use in an aerosol dispenser and other containers and dispensers designed to provide creams, lotions, sprays, foams, gels and the like under pressure. The microemulsion of the present invention provides an aerosol product that has the appearance and performance of a uniform, single phase.

The preferred propellant of the present invention does not harm the ozone layer and has a reduced impact on global warming. The propellant may have an ozone depletion potential of less than or equal to 0.05 and a global warming potential of less than or equal to 1000, or, more specifically, less than or equal to 750, less than or equal to 500 or, even more specifically, less than or equal to 150.

### The Water Soluble Polymer

The preferred compositions comprise a water soluble, preferably hydoxy-containing polymer. Preferred water soluble polymers include water soluble polyethers. Preferred water soluble polyethers include water soluble polyakylene glycols, including preferable polyethylene glycols, water soluble polypropylene glycols, and mixtures thereof.

In highly preferred embodiments, the water soluble polypropylene glycol (PPG) is a PAG having a molecular weight of under about 2000, more preferably under about 1000, and even more preferably less than about 500, with molecular weight of from about 350 to about 450. In preferred embodiments, the water soluble polyether comprises polypropylene glycol having a molecular weight of about 400. Preferably, the preferred PPG has a hydroxyl number of from about 240 to about 280. Preferably, the preferred PPG has a hydroxyl number of from about 240 to about 280. One preferred PPG having a molecular weight of about 400 and a hydroxyl number of from about 240 to about 280 is the product sold under the trade name JEFFOX PPG-400 by Hunstman.

The water soluble polyether preferable has a molecular weight under about 2000, and more preferably under about 1000. In preferred embodiments, the water soluble polyether comprises polypropylene glycol 400.

The water soluble polymer, and particularly polyethylene glycol and/or polypropylene glycol, may be present in a wide variety of amounts, depending of the needs of a particular application. In preferred embodiments, the water soluble polymer is present in the microemulsion of the present invention in an amount from about 10 % to about 60 % by weight of the microemulsion.

### The Hydrotrop

As mentioned above, an important component of the present microemulsions is a specific form of an ionic surfactant known as a hydrotrops. Hydrotrops consists generally of two parts, an anionic group, preferably for this invention a carboxylate or sulfonate, and a hydrophobic group, preferably for this invention an aromatic ring or ring system. Preferred hydrotrops include salts of xylene sulfonate, para-toluene sulfonate, cumene sulfonate, and the like. The salts may comprise sodium, potassium, ammonium, or the like. A particularly preferred hydrotrop for use in the stable micro-emulsion composition of the present invention is sodium xylene sulfonate.

The hydrotrops, such as sodium xylene sulfonate, may be present in a wide variety of amounts, depending of the needs of a particular application. In preferred embodiments, the hydrotrope is present in an amount of from about 4 % to about 25 % by weight of the micro-emulsion composition, and even more preferably in an amount of from about 5 % to about 15 % by weight of the micro-emulsion composition.

### Optional Components

### Co-Solvents

The compositions of the present invention may further comprise a co-solvent which can enhance and help to provide improvement in one or more qualities of the micro-emulsion. Preferred co-solvents may include alcohols, glycols including glycol ethers, and mixtures thereof. Preferred alcohols include ethanol, propanol, iso-propanol, glycerin, propanediol, 1-propoxy -2-propanol, 1-butoxy -2-propanol, and the like, and mixtures thereof. Preferred glycols include dipropylene glycol n-butyl ether, dipropylene glycol n-propyl ether, diethylene glycol butyl ether, dipropylene glycol monomethyl ether, ethylene glycol monopropyl ether, diethylene glycol monomethyl ether, diethylene glycol monoethyl ether, triethyleneglycol monobutyl ether, propylene glycol monomethyl ether, triethylene glycol monomethyl ether, tripropylene glycol monobutyl ether, and the like.

### Surfactants

It is contemplated that one or more surfactants, other than the hydrotrop, may be included in some embodiments, although it is in many embodiments not preferred. Accordingly, in some applications it is contemplated that the present micro-emulsions will not include any substantial amount of a co-surfactant. When a co-surfactant is included, it is contemplated that it may comprise a non-ionic surfactant, an ionic surfactant, or a mixture thereof. In certain embodiments, the emulsifying composition comprises a non-ionic surfactant and an ionic surfactant.

Non-ionic surfactants for use in the composition can include if needed for a particular application polyethers and ethoxylated alcohols, and mixtures thereof. Polyethers include polyethylene glycol, polypropylene glycol, copolymers of polyethylene glycol and polypropylene glycol, and mixtures thereof. In some embodiments, the non-ionic surfactant comprises polypropylene glycol. In some embodiments, the polyethers and ethoxylated alcohols may alternatively or additionally perform as solvents in the composition.

### Preferred Formulations

The compositions of the present invention form stable emulsions, and preferably form stable microemulsions. The microemulsions are ideally clear, thermodynamically stable, liquid mixtures of the HFO-1234 propellant, water and surfactants. In contrast to ordinary emulsions, the microemulsions preferably form upon simple mixing of the components and do not require the high shear conditions generally used in the formation of ordinary emulsions.

In preferred embodiments, the microemulsion is stable upon standing. It is particularly beneficial if the aqueous microemulsion composition comprising is stable when added to ingredients used to make the final composition. Stability of the formulated microemulsion solution were done at 40 OC in an oven for six weeks. All formulations were found to be stable after thermal stability test.

The compositions may also contain optional additional ingredients, including surfactants, co-solvents, rheology modifiers, pH modifier, buffers, dyes, fragrances, etc.

Accordingly, a preferred embodiment of the present invention provides an aqueous micro-emulsion that comprises:
a. from about 1% to about 20% by weight of water, more preferably from about 4% to about 15% by weight of water, and even more preferably from about 4% to about 15% by weight of water;
b. a water soluble, hydroxy-containing polymeric material, preferably comprising a polyalkyleneglycol, and even more preferably polyethylene glycol (PEG) and/or polypropyleneglycol (PPG), and preferably present in an amount of from about 20% % to about 70% by weight, more preferably from about 40% % to about 60% by weight, more preferably from about 40% % to about 50% by weight;
c. at least one hydrotrop, preferably present in an amount of from about 2% % to about 15% by weight, more preferably from about 2% % to about 10% by weight; and
d. a propellant comprising trans-1,3,3,3-tetrafluoropropene (HFC-1234ze(E) or transHFO-1234ze), in an amount of from about 5% % to about 65% by weight, more preferably from about 10 % to about 50% by weight, more preferably from about 10% % to about 40% by weight, wherein said polypropylene glycol and said at least one hydrotrop are present in amounts effective to create a stable microemulsion of said propellant in said water or said water in said propellant, and wherein said aqueous microemulsion contains no substantial amount of an oil phase dispersed in said aqueous continuous phase.

### THE APPLICATION COMPOSITIONS

The aqueous micro-emulsion composition may be used as part of the composition for water-based aerosol products, including cosmetics, personal care products, pharmaceuticals, household cleaners, and in surface coating applications. In such applications, the micro-emulsion of the present invention will further contain an active component that is included for the purpose of effecting a particular purpose when delivered to the surface of the article or body to be treated. It is contemplated that a wide variety of such active components are useful in connection with the present micro-emulsions and included within the scope hereof.

### THE DEVICES AND METHODS

The present invention also provides devices and containers, and preferably pressurized containers, that contain the present micro-emulsions and from which the present micro-emulsion is dispensed when and at the location of intended use. The container will normally include a nozzle, orifice or the like for maintaining the container in a normally closed positon but which can be activated/opened to allow the micro-emulsion to escape, to be poured, to be sprayed and the like to the location of use.

The present devices and methods thus provide an advantage in that the micro-emulsion contains a propellant, which upon opening or activating the nozzle or opening of the container, will provide a motive force to the move the microemulsion and/or other container contents out of the container, such as a pressurized can or the like. In certain preferred embodiments, the present aqueous micro-emulsion is contained in an aerosol container,

### EXAMPLES

### Comparative Example 1

The following combinations of know surfactants were prepared to achieve a variety of different HLB values, and then each of these combinations were evaluated for the ability to form a stable emulsion of transHFO-1234ze and water.

**TABLE CE1**

| Comarative Example | Span-80 (g) | Span-85 (g) | Tween-80 (g) | Tween-20 (g) | D.I. water (g) | HLB |
|---|---|---|---|---|---|---|
| C1 | 0.34 | 3.68 | Nil | Nil | 95.99 | 2 |
| C2 | 3.51 | 0.50 | Nil | Nil | 95.99 | cC4 |
| C3 | 3.35 | Nil | 0.66 | Nil | 95.99 | 6 |
| C4 | 2.57 | Nil | 1.42 | Nil | 96.01 | 8 |
| C5 | 1.86 | Nil | 2.20 | Nil | 95.94 | 10 |
| C6 | 1.12 | Nil | 2.87 | Nil | 96.01 | 12 |
| C7 | 0.37 | Nil | 3.66 | Nil | 95.97 | 14 |
| C8 | Nil | Nil | 1.62 | 2.41 | 96.0 | 16 |

Each of the surfactant solution (50.0 g) was used to mix with 5.0 g of trans-1234ze in a Fischer-Port tube. Gas pressure in the tube was recorded with a pressure gauge. 5.0 g of trans-1234ze in the solution mixture provided 60 psi of gas pressure. Applicants found that none of the tested surfactants were able to form a stable, clear mixture of trans-1234ze with water.

### Comparative Example 2

Attempts to form micro-emulsions using certain mixtures of surfactants was attempted by forming the following mixtures. In each case the liquid components were prepared under ambient temperatures and pressures, and then the propellant transHFO-1234ze was added to the liquid under sufficient pressure to maintain the transHFO-1234ze in liquid form. The following Table CE2 provides the mixtures and the observed results:

**TABLE CE2**

| Experiment # | Formulation Composition | Remarks |
|---|---|---|
| CE9 | 7.0 ml. of sodium xylene sulfonate + 4.0 ml. of 1234ze (60 psi) | **Bubble formed and separated slowly into two phases** |
| CE10 | 7.0 ml. sodium xylene sulfonate + 4.0 ml. 1234ze + 2.5 ml. of a mixture of (Span -80 + span-85) (HLB=2) | **Bubble formed and separated slowly into two phases** |
| CE11 | 5.0 ml. sodium xylene sulfonate + 5 ml. 1234ze (60 psi) + 2.5 ml. surfactant mixture of ( Tween 20 + Tween 80) with HLB=16 | **Bubble formed and separated slowly into two phases** |

### Comparative Example 3

In each of these comparative examples, an initial liquid solution is prepared comprising to form an aqueous solution containing 40% by weight of sodium xylene sulfonate (60% by weight of water). The hydrotrops identified in the Table C3 below were then added, together with any other components having a normal boiling point below room temperature.

**TABLE CE3**

| Comparative Example | Experimental Conditions | Remarks |
|---|---|---|
| CE12 | 10.01 g. 1234ze + 5.01 g. D.I. water + 6.3 g PEG (polyethylene Glycol) | Milky Emulsion, slowly breaks down into two phases |
| CE13 | 5.01 g. water + 5.01 g. PEG + 11.8 g, 1234ze | Emulsion breaks down |
| CE14 | 10.03 g. water + 10.02 g. PEG + 3.72 g. 1234ze | Unstable emulsion |
| CE15 | 10.01 g. water + 5.18 g Sodium xylene sulfonate + 7.1 g 1234ze | Phase separation |
| CE16 | 5.03 g. water + 5.06 g. sodium cumene sulfonate + 11.3 g. 1234ze | Phase separation |
| CE17 | 10.08 g. water + 5.18 g. sodium xylene sulfonate + 5.53 g. 1234ze | Phase separated |
| CE18 | 5.06 g. water + 5.07 g. sodium xylene sulfonate + 11.29 g. 1234ze | Phase separated |
| CE19 | 10.03 g. water + 5.05 g. Propylene Glycol (P-400) + 6.24 g. 1234ze | Phase separated |
| CE20 | 5.01 g. water + 5.01 g. PPG - 400 + 11.27 g. 1234ze | Phase separated |
| CE21 | 10.01 g. water + 10.02 g. PPG-400 + 7.32 g 1234ze | Phase separated |
| CE22 | 7.34 g. sodium xylene sulfonate + 6.78 g. 1234ze | Phase separated |
| CE23 | 5.03 Polyethylene glycol + 4.92 g. 1234ze | Miscible (Hazy) |
| CE24 | 2.55 g. Polyethylene glycol + 2.55 g. polypropylene glycol + 5.12 g. 1234ze | Phase separated |
| CE25 | 10.06 g. water + 5.05 g. PEG (polyethylene Glycol) + 1.03 g. Ethanol + 4.86 g. 1234ze | Phase separated |
| CE26 | 10.02 g. water + 5.03 g. PPG-400 + 1.05 g. ethanol + 5.62 g. 1234ze | Phase separated |
| CE27 | 10.03 g. water + 7.03 g. sodium xylene sulfonate + 6.58 g. 1234ze | Phase separated |
| CE28 | 5.14 g. sodium xylene sulfonate + 5.02 g. PPG-400 + 3.23 g. 1234ze | Phase separated |
| CE29 | 10.01 g. sodium xylene sulfonate + 5.01 g. PPG-400 + 3.72 g. 1234ze | Phase separated |
| CE30 | 10.06 g. PPG-400 + 5.05 g. sodium Xylene sulfonate + 3.22 g. 1234ze | Hazy solution |
| CE31 | 5.01 g. Polyethylene glycol + 5.23 g. 1234ze | Not miscible |
| CE32 | 0.07 g Toluene sulfonate + 5.02 g. 1234ze | Phase separated |
| CE33 | 10.0 g. 1234ze + 5.0 g. PEG + 5.0 g PPG-400 + 5.0 g. Sod. Xylene sulfonate | Phase separated |
| CE34 | 5.02 g. PPG-400 + 5.02 g. Sodium xylene sulfonate | Not miscible |
| CE35 | 5.03 g. PPG-400 2.53 g. sodium xylene sulfonate + 1.02 g. Diisopropyl amine | Light hazy emulsion |
| CE36 | 5.05 g. PPG-400 + 2.27 g. sodium xylene sulfonate + 1 drop of Span 80 | Hazy emulsion |
| CE37. | 2.01 g. PPG-400 + 1.0 g. sodium xylene sulfonate + 1 drop of span 85 | Hazy emulsion |
| CE38 | 2.53 g. sodium xylene sulfonate + 5.01 g. PPG-400 + 4.77 g. 1234ze | Phase separation |
| CE39 | 2.54 g. sodium xylene sulfonate + 5.01 g. PPG-400 + 0.3 g. Isopropanol + 3.3 g. 1234ze | Phase separation |
| CE40 | 10.02 g. PPG-400 + 3.01 g. Sodium xylene sulfonate + 2.41 g. 1234ze | Unstable, lower level - diffused, upper level-clear |
| CE41 | 1.59 g. sod. Xylene sulfonate + 5.12 g. PPG-400 + 1.58 g. Glycerin + 2.05 g. 1234ze | Unstable, both layers are clear |
| CE42 | 1.52 g. Sod. Xylene sulfonate + 1.55 g. glycerin + 5.02 g. PPG-400 + 3.58 g. 1234ze | unstable |
| CE43 | 5.02 g. PPG-400 + 1.56 g. sod. Xylene sulfonate + 1.58 g. glycerin + 0.77 g. 1234ze | unstable |
| CE44 | 1.55 g. sod. Xylene sulfonate + 1.53 g. glycerin + 5.01 g. PPG-400 = 0.22 g. Isopropanol + 4.01 g 1234ze | unstable |

### Example 1. Stable, Aqueous Micro-emulsification of trans1234ze

Micro-emulsions were formed using the following procedures. In each case the liquid components of the formulation in the following Table E1 (water, PEG, sodium xylene sulfate, PPG 400, etc) when present, were prepared under ambient temperatures and pressures, and then the propellant transHFO-1234ze was added to the liquid under sufficient pressure to maintain the transHFO-1234ze in liquid form. The mixture was then observed for at least sic (6) weeks and found to have the indicated formation of a stable, clear micro-emulsion.

| Example | Experimental conditions | Remarks |
|---|---|---|
| 42B. | 5.02 g. PPG-400 + 1.56 g. Sod. Xylene sulfonate + 1.56 g. Glycerin + 2.56 g. 1234ze | **Clear solution** |
| 43B. | 1.50 g. Sod. Xylene sulfonate + 5.12 g. PPG-400 + 1.56 g. Glycerin + 2.05 g. 1234ze | **Clear solution** |
| 44B. | 5.02 g. PPG-400 + 1.56 g. Sod. Xylene sulfonate + 1.58 g. Glycerin + 0.77 g. 1234ze | **Clear solution** |
| 45B. | 1.52 g. sod. Xylene sulfonate + 1.55 g. Glycerin + 5.02 g. PPG-400 + 13.57 g. 1234ze | **Very Clear solution** |
| 46. | 10.0 g. PPG-400 + 5.03 g. Sod. Xylene sulfonate + 3.22 g 1234ze | **Very clear, stable, one phase** |
| 47. | 5.03 g. PPG-400 + 2.53 g. Sod. Xylene sulfonate + 0.32 g. Isopropanol + 3.48 g. 1234ze | **Very clear, stable, one phase** |
| 48. | 5.03 g. PPG-400 + 2.53 g. Sod. Xylene sulfonate + 0.3 g. Isopropanol + 3.30 g. 1234ze | **Stable, one phase, clear** |
| 49. | 2.52 g. Sod. Xylene sulfonate + 0.3 g. Isopropanol + 5.01 g PPG-400 + 3.14 g. 1234ze | **Very Clear, Stable, One phase** |
| 51. | 5.01 g. Sod. Xylene sulfonate + 10.02 g. PPG-400 + 5.05 g. PPG-400 + 2.16 g. 1234ze | **Very clear, stable, one phase** |
| 52. | 2.59 g. Sodium xylene sulfonate + 5.04 g. PPG-400 + 3.96 g. 1234ze | **Very clear, stable, one phase** |
| 53 | 5.02 g. PPG-400 + 5.13 g. sodium xylene sulfonate | **Miscible and transparent** |
| 54 | 3.02 g Diisopropyl amine + 7.45 g. 1234ze | **Miscible and transparent** |
| 55 | 5.01 Polypropylene glycol (P-400) + 4.56 g. 1234ze | **Soluble (clear)** |
| 56 | 5.02 g. PPG-400 + 1.56 g. sod. Xylene sulfonate + 1.56 g. Glycerin + 2.56 g. 1234ze | **Clear solution** |

### Clauses

1. A stable micro-emulsion comprising:
   (a) from about 1% to about 20% by weight of water;
   (b) a water soluble, hydroxy-containing polymeric material in an amount of from about 20% % to about 70% by weight;
   (c) at least one a hydrotrop present in an amount of from about 2% % to about 15% by weight; and
   (d) a propellant comprising trans-1,3,3,3-tetrafluoropropene (HFC-1234ze(E) or transHF0-1234ze), in an amount of from about 5% % to about 65% by weight, wherein said weight percentages are based on said components (a) - (d) and wherein said water soluble, hydroxy-containing polymeric material and said at least one hydrotrop are present in amounts effective to create a stable microemulsion of said propellant in said water or said water in said propellant.
2. The stable micro-emulsion of clause 1 comprising from about 4% to about 15% by weight of water.
3. The stable micro-emulsion of clause 1 comprising from about 4% to about 15% by weight of water.
4. The stable micro-emulsion of any of clauses 1 - 3 wherein said water soluble, hydroxy-containing polymeric material comprises a polyalkyleneglycol.
5. The stable micro-emulsion of any of clauses 1 - 3 wherein said water soluble, hydroxy-containing polymeric material consistes essentially of polyalkyleneglycol.
6. The stable micro-emulsion of any of clauses 1 - 3 wherein said water soluble, hydroxy-containing polymeric material consists of polyalkyleneglycol.
7. The stable micro-emulsion of any of clauses 1 - 3 wherein said water soluble, hydroxy-containing polymeric material is selected from polyethylene glycol (PEG) and/or polypropyleneglycol (PPG).
8. The stable micro-emulsion of any of clauses 1-7 wherein said water soluble, hydroxy-containing polymeric material has a molecular weight of from about 350 to about 450.
9. The stable micro-emulsion of any of clauses 1-7 wherein said water soluble, hydroxy-containing polymeric material has a hydroxyl number of from about 240 to about 280.
10. The stable micro-emulsion of any of clauses 1 - 9 wherein said water soluble, hydroxy-containing polymeric material comprises polypropyleneglycol (PPG).
11. The stable micro-emulsion of any of clauses 1 - 9 wherein said water soluble, hydroxy-containing polymeric material consists essentially of polypropyleneglycol (PPG).
12. The stable micro-emulsion of any of clauses 1-11 wherein said water soluble, hydroxy-containing polymeric material is present in an amount of from about 40% % to about 60% by weight.
13. The stable micro-emulsion of any of clauses 1-11 wherein said water soluble, hydroxy-containing polymeric material is present in an amount of from about 40% % to about 50% by weight.
14. The stable micro-emulsion of any of clauses 1 - 13 wherein said hydrotrop is present in an amount of from about 2% % to about 10% by weight.
15. The stable micro-emulsion of any of clauses 1 - 13 wherein said hydrotrop is present in an amount of from about 5% to about 15% by weight.
16. The stable micro-emulsion of any of clauses 1-16 wherein said hydrotrop consists of an anionic group selected from a carboxylate or sulfonate, and a hydrophobic group selected from an aromatic ring or ring system.
17. The stable micro-emulsion of any of clauses 1-16 wherein said hydrotrop is a salt of xylene sulfonate.
18. The stable micro-emulsion of any of clauses 1-16 wherein said hydrotrop is sodium xylene sulfonate.
19. The stable micro-emulsion of any of clauses 1-16 wherein said hydrotrop comprises sodium xylene sulfonate.
20. The stable micro-emulsion of any of clauses 1-16 wherein said hydrotrop is a salt of xylene sulfonate and/or para-toluene sulfonate and/or cumene sulfonate.
21. The stable micro-emulsion of clause 20 wherein said salt is a sodium, potassium or , ammonium salt.
22. The stable micro-emulsion of any of clauses 1 - 21 wherein said trans-1,3,3,3-tetrafluoropropene (HFC-1234ze(E) is present said micro-emulsion in an amount of from about 10 % to about 50% by weight.
23. The stable micro-emulsion of any of clauses 1 - 21 wherein said trans-1,3,3,3-tetrafluoropropene (HFC-1234ze(E) is present said micro-emulsion in an amount of from about 10 % to about 30% by weight.
24. The stable micro-emulsion of any of clauses 1 - 23 wherein the propellant comprises transHF0-1234ze in an amount of at least about 75 % by weight based on the total propellant.
25. The stable micro-emulsion of any of clauses 1 - 23 wherein the propellant comprises transHF0-1234ze in an amount of at least about 80 % by weight based on the total propellant.
26. The stable micro-emulsion of any of clauses 1 - 23 wherein the propellant comprises transHF0-1234ze in an amount of at least about 85 % by weight based on the total propellant.
27. The stable micro-emulsion of any of clauses 1 - 23 wherein the propellant comprises transHF0-1234ze in an amount of at least about 90 % by weight based on the total propellant.
28. The stable micro-emulsion of any of clauses 1 - 23 wherein the propellant comprises transHF0-1234ze in an amount of at least about 95 % by weight based on the total propellant.
29. The stable micro-emulsion of any of clauses 1 - 24 wherein the propellant comprises transHF0-1234ze in an amount of at least about 99.5 % by weight based on the total propellant.
30. The stable micro-emulsion of any of clauses 1-29 wherein wherein said aqueous microemulsion contains no substantial amount of an oil phase.
31. The stable micro-emulsion of any of clauses 1 - 30 wherein wherein said aqueous microemulsion is a propellant in water emulsion containing no substantial amount of an oil phase.
32. The stable micro-emulsion of any of clauses 1 - 30 further comprising glycerin, isopropano1, or a mixture thereof.

## Claims

1. A stable micro-emulsion comprising:
(a) from about 1% to about 20% by weight of water;
(b) a water soluble, hydroxy-containing polymeric material in an amount of from about 20% to about 70% by weight;
(c) at least one a hydrotrop present in an amount of from about 2% to about 15% by weight; and
(d) a propellant comprising trans-1,3,3,3-tetrafluoropropene (HFO-1234ze(E) or transHF0-1234ze), in an amount of from about 5% to about 65% by weight, wherein said weight percentages are based on said components (a) - (d) and wherein said water soluble, hydroxy-containing polymeric material and said at least one hydrotrop are present in amounts effective to create a stable microemulsion of said propellant in said water or said water in said propellant.

2. The stable micro-emulsion of claim 1 comprising from about 4% to about 15% by weight of water.

3. The stable micro-emulsion of claim 1 wherein said water soluble, hydroxy-containing polymeric material consists essentially of polyalkyleneglycol.

4. The stable micro-emulsion of claim 1 wherein said water soluble, hydroxy-containing polymeric material is selected from polyethylene glycol (PEG) and/or polypropyleneglycol (PPG).

5. The stable micro-emulsion of claim 1 wherein said water soluble, hydroxy-containing polymeric material has a molecular weight of from about 350 to about 450.

6. The stable micro-emulsion of claim 5wherein said water soluble, hydroxy-containing polymeric material comprises polypropyleneglycol (PPG).

7. The stable micro-emulsion of claim 6 wherein said water soluble, hydroxy-containing polymeric material is present in an amount of from about 40% to about 60% by weight.

8. The stable micro-emulsion of claim 7 wherein said hydrotrop is present in an amount of from about 2% to about 10% by weight.

9. The stable micro-emulsion of claim 1 wherein said hydrotrop consists of an anionic group selected from a carboxylate or sulfonate, and a hydrophobic group selected from an aromatic ring or ring system.

10. The stable micro-emulsion of claim 9 wherein said hydrotrop is a salt of xylene sulfonate and/or para-toluene sulfonate and/or cumene sulfonate.

11. The stable micro-emulsion of claim 1 wherein said trans-1,3,3,3-tetrafluoropropene (HFO-1234ze(E)) is present said micro-emulsion in an amount of from about 10 % to about 50% by weight.

12. The stable micro-emulsion of claim 1 wherein the propellant comprises transHF0-1234ze in an amount of at least about 75 % by weight based on the total propellant.

13. The stable micro-emulsion of claim 1 wherein the propellant comprises transHF0-1234ze in an amount of at least about 95 % by weight based on the total propellant.

14. The stable micro-emulsion of claim 1 wherein said aqueous microemulsion is a propellant in water emulsion containing no substantial amount of an oil phase.

15. The stable micro-emulsion of claim 1 further comprising glycerin, isopropanol, or a mixture thereof.
